# EUROPEAN PATENT SPECIFICATION

(11) **EP 1 460 077 B1**
(45) Date of publication and mention of the grant of the patent: **01.08.2007**
(21) Application number: 04006048.5
(22) Date of filing: 15.03.2004
(51) Int. Cl.: C07D 487/04, A61K 31/519, A61P 15/10, C07D 473/30, A61K 31/496, C07D 239/00, C07D 231/00

(54) **Novel pyrazolopyrimidones and their use as PDE inhibitors**
Pyrazolopyrimidinone und ihre Verwendung als PDE inhibitoren
Pyrazolopyrimidinone et leur application comme inhibiteurs de la PDE

(30) Priority: 18.03.2003 DE 10312021; 04.02.2004 DE 10405392
(43) Date of publication of application: 22.09.2004
(73) Proprietor: The Jordanian Pharmaceutical Manufacturing Co. Ltd., 11710 Naor (JO)
(72) Inventor: Badwan, Ali Adnan, 11710 Naor (JO)
(74) Representative: Winkler, Andreas Fritz Ernst

(56) References cited:
- EP-A- 0 201 188
- EP-A- 1 022 026
- EP-A- 1 219 614
- US-A- 3 939 161
- TERRETT N K ET AL: "Sildenafil (VIAGRA<TM>), a potent and selective inhibitor of type 5 CGMP phosphodiesterase with utility for the treatment of male erectile dysfunction" BIOORGANIC & MEDICINAL CHEMISTRY LETTERS, OXFORD, GB, vol. 6, no. 15, 6 August 1996 (1996-08-06), pages 1819-1824, XP004135609 ISSN: 0960-894X

## Description

The invention relates to novel compositions comprising pyrazolopyrimidones as well as to the use of the compositions for the preparation of a medicament.

Substituted pyrazolopyrimidones are known in the prior art, in particular as anti-convulsants, sedatives, and anti-inflammatory and gastric antisecretory agents (US patent No. 3,939,161) and for use in the treatment of cardiovascular disorders (US patent No. 4,666,908).

Early generation of PDE inhibitors exhibited pharmacological effects on more than one PDE. This is due to vast distribution of PDEs in different organs in the body and the lack of fully understanding the mechanism of such inhibitors. Although PDE inhibitors are claimed to act more selectively on one PDE family, but their pharmacological action is shown to act on more than a single site (organ). This can be attributed to their structure, which is usually a combination of two active parts.

The treatment of erectile dysfunction is undergoing a tremendous advancement. Three major medicaments as phosphodiesterase inhibitors, namely sildenafil, tadalafil and vardenafil, are commercially available. From these compounds, it is known that they show significant toxicity and side-effects

EP 1 022 026 A2 discloses methods for treating nitrate-induced tolerance in a mammal by administering a nitrate-induced tolerance treating amount of a specific compound. Also disclosed are pharmaceutical compositions for the treatment of nitrate-induced tolerance in a mammal comprising a nitrate-induced tolerance treating amount of this compound.

N.K. Terrett, A.S. Bell, D. Brown and P. Ellis, "Sildenafil, a potent and selective inhibitor of type 5 cGMP phosphodiesterase with utility for the treatment of male erectile dysfunction" Bioorganic & Medicinal Chemistry Letters, Volume 6, No. 15, pp. 1819-1824, 1996, disclose 5-(2'-alkoxyphenyl)pyrazolo[4,3-d]pyrimidin-7-ones which are portent and selective inhibitors of the type 5 cGMP phosphodiesterase from both rabbit platelets and human corpus cavernosum.

EP 0 201 188 A1 discloses 5-substituted pyrazolo[4,3-d]pyrimidin-7-one compounds, compositions, methods of use and processes to make the compounds. These compounds are useful in the treatment of cardiovascular disorders, such as heart failure or cardiac insufficiency. The compounds bind adenosine receptors and selectively inhibit phosphodiesterase.

It is an object of the present invention to provide novel compositions which might be used for the production of a medicament acting as PDE inhibitor, such as for the treatment of erectile dysfunction, which has a sufficient activity and shows decreased toxicity and side-effects as the presently known medicaments.

This object is achieved by the novel compositions as disclosed in claim 1 and 2 as well as the uses of claims 8 and 9.

Further preferred embodiments are disclosed in the sub-claims. Compound B is disclosed in EP 1219 614 A1.

Surprisingly, it was found that the action of composition of the present invention on tissues containing PDE are higher compared to compounds known in the prior art. Especially, the inventive compositions show a synergistic action on tissues containing PDEs, the action being higher than either one molecular entity or if both parts are jointly together in one compound.

Surprisingly, it was also found that compositions of the present invention are especially active for the treatment of erectile dysfunction with a strongly improved efficiency. Experiments have shown that those compositions are at least as active and more favorable as to toxicity and side-effects as the presently most widely used medicament for the treatment of erectile dysfunction, namely sildenafil (Viagra^{®}).

Presently, best results are obtained, for compositions with a compound (A) wherein R₁ and R₂ are both hydrogen, R₃ is methyl and R₄ is n-propyl. Also, it is preferred for the compound (B) of the composition that X is N, R₀ is H, methyl, methoxy or ethoxy.

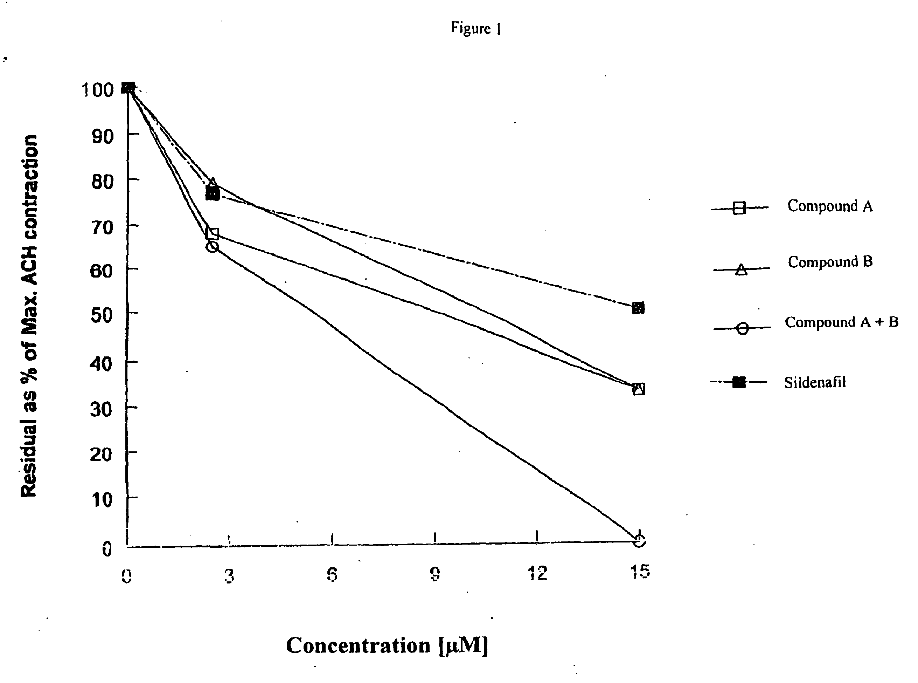

The surprising effect achieved by the present invention is now further illustrated in the examples section with reference to the accompanying drawing (Figure 1) which shows for a single compound A, a single compound B, a mixture of compounds A and B and sildenafil the dependency of residual of maximum ACH contraction (%) on the concentration of the compounds. Compound A used has the structure of formula (III), wherein R₁ and R₂ are both hydrogen, R₃ is methyl and R₄ is n-propyl. As can be clearly seen from the figure, the mixture of both compounds A and B shows superior activity than for each compound alone and even superior activity than for sildenafil.

Further, the effect of administration of above compositions was studied by administrating them to both rats and rabbits.

The purpose of the study was to compare the biological activity of the compositions of the present invention with that of sildenafil. In particular, the erection episodes and penile erection indexes of the compounds and compositions in the treatment of male rats and rabbits were determined.

The Penile Erection Index (PEI) is calculated or expressed as the % of rats or rabbits exhibiting at least one episode of penile erection multiplied by the number of total episodes, within a time period of 2 hours. Details on the determination of PEI values can be found in e.g. H.H. Ang and M.K. Sim, Effects of Eurycoma longifilia Jack on Penile Erection Indes and Homosexual Mounting in Rats, Pharmaceutical Sciences, 3 (1997), 117-19; and A. Benassi-Benelli, F. Ferrari and B. Pellegrini Quaratotti, Penile erection Induced by Apomorphine and N-n-propyl-norapomorphine in Rats, Arch. Int. Pharmacodyn 242 (1979), 241-247. The episode is a description for rat sucking their penus.

Rabbit experiments where performed according to E. Bischof and K. Schneider, "International Journal of Impotence Research", 13 (2001) 230 - 235, and rat experiments where performed according to EP 1057829 A1.

Studying the penile erection index for rabbits in dependency on time yields the results given in table 1 for sildenafil and the compound (III).

**Table 1**

| Time (min) | Sildenafil | Compound III |
|---|---|---|
| 0 | 0 | 0 |
| 5 | 18.318 | 12.8 |
| 10 | 16.694 | 9.5 |
| 15 | 15.22 | 7.4 |
| 20 | 13.108 | 5 |
| 30 | 11.218 | 1.8 |
| 40 | 7.364 | 1.8 |
| 50 | 2.94 | 1 |

The data of table 1 show that sildenafil has greater efficacy than compounds III. However, the surprising effect is that using a composition as disclosed in the present invention leads to almost similar effect as that of sildenafil, but at optimum concentration and mixing ratios the side effect of the medicament based on the compositions of the present invention is less than for sildenafil. Of course, the ratio will be dependent on the target medication, for example erectile dysfunction or asthma treatment, etc..

Further, the effect on penile length of rats was studied and the following results are given in table 2 for a compound disclosed in the invention and in table 3 for sildenafil. The penile length is indicative of the efficacy of the administered medicament.

**Table 2**

| Compound III | | | |
|---|---|---|---|
| dose mg/kg | No. of rats | No. of episodes | PEI |
| Vehicle | 10 | 0 | 0 |
| 2 | 10 | 7 | 280 |
| 5 | 10 | 13 | 910 |
| 10 | 10 | 8 | 400 |
| 20 | 10 | 9 | 270 |

**Table 3**

| Sildenafil | | | |
|---|---|---|---|
| dose mg/kg | No. of rats | No. of episodes | PEI |
| Vehicle | 10 | 0 | 0 |
| 0.0781 | 10 | 3 | 90 |
| 0.1562 | 10 | 5 | 150 |
| 0.3125 | 10 | 7 | 350 |
| 0.6250 | 10 | 14 | 980 |

Features disclosed in the description, in the claims and in the drawing may, both separately and in any combination thereof, be material for realizing the invention in diverse forms thereof.

## Claims

1. Composition comprising a mixture of compound (A) represented by one of the structural formulas: 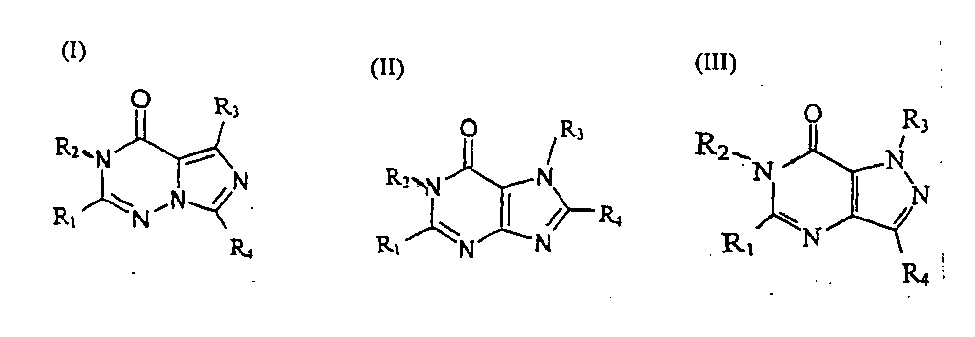 wherein R₁, R₂, R₃, and R₄ are independently hydrogen, halogen, hydroxyl, amino, alkyl, alkenyl, alkynyl, cycloalkyl, cycloalkenyl, cycloalkynyl, haloalkyl, alkylaryl, aryl, aralkyl, alkoxy, carboxy or heterocyclyl, all of these substituents being substituted or unsubstituted, with the exception of formula (III), wherein R₁ being hydrogen, C₁-C₄ alkyl, C₁-C₄ haloalkyl, piperidinomethyl, methoxymethyl, N-methylpiperazino methyl, carbethoxy, p-chlorophenoxymethyl or Ar-(CH₂)n-, wherein n is 0-4; and Ar is 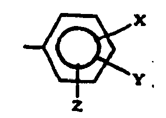 or 2, 3, or 4-pyridyl, wherein X, Y, and Z are independently (1) hydrogen; (2) lower alkyl of from one to six carbons, inclusive; (3) halogen; (4) hydroxyl; (5) lower alkoxy of from one to six carbons, inclusive; (6) nitro; (7) amino; (8) NR'R" wherein R' and R" are each independently (a) hydrogen or (b) lower alkyl of from one to six carbons, inclusive, optionally substituted by (i) amino, (ii) morpholino or (iii) cycloalkyl of from five to seven carbons, inclusive; (9) sulfo; or (10) -SO₂NR'R" wherein R' and R" are as defined above with the proviso that not all of X, Y, and Z can be nitro, amino, or NR'R" at once; and R₂ being hydrogen, C₁-C₄ alkyl, phenyl or 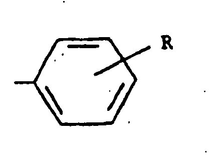 wherein R is a substituent of the group consisting of halo, methyl, trifluoromethyl and di(C₁-C₄)alkylamino (C₁-C₄)alkyloxy; when R₃ and R₄ are both methyl, and pharmaceutically acceptable salts thereof,
and of compound (B) represented by the structural formula: 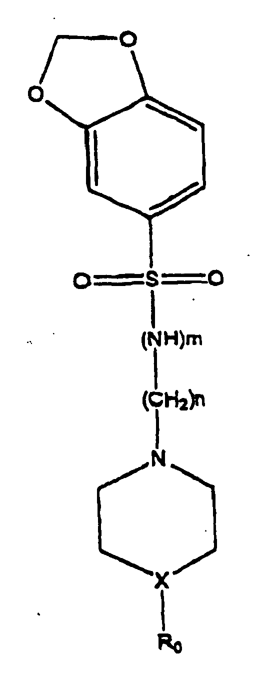 wherein
X is chosen from N and C;
R₀ is chosen from H, a lower alkyl group having 1 to 6 carbon atoms, a lower O-alkyl group having 1 to 6 carbon atoms, a lower aldehyde group having 1 to 6 carbon atoms, a benzyl group, a phenyl group, a phenyl group substituted with halogen or O-alkyl having 1 to 6 carbon atoms, a heterocyclic amine group having 3 to 6 carbon atoms, a cycloalkyl group having from 3 to 6 carbon atoms, a cycloalkyl group having from 3 to 6 carbon atoms substituted with O-alkyl having 1 to 6 carbon atoms, and a furyl group;
n is 0, 1, 2, 3 or 4;
m is 0 or 1;
and pharmaceutically acceptable salts thereof

2. Composition comprising a mixture of compound (III) 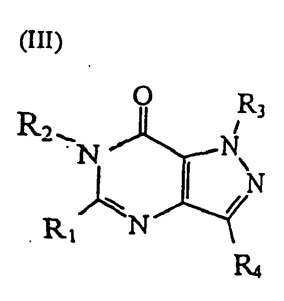 wherein R₁ and R₂ are both hydrogen, R₃ is methyl and R₄ is n-propyl,
and of compound (B) represented by the structural formula: 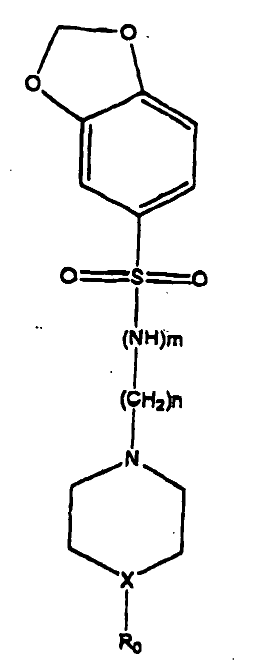 wherein
X is chosen from N and C;
R₀ is chosen from H, a lower alkyl group having 1 to 6 carbon atoms, a lower O-alkyl group having 1 to 6 carbon atoms, a lower aldehyde group having 1 to 6 carbon atoms, a benzyl group, a phenyl group, a phenyl group substituted with halogen or O-alkyl having 1 to 6 carbon atoms, a heterocyclic amine group having 3 to 6 carbon atoms, a cycloalkyl group having from 3 to 6 carbon atoms, a cycloalkyl group having from 3 to 6 carbon atoms substituted with O-alkyl having 1 to 6 carbon atoms, and a furyl group;
n is 0, 1, 2, 3 or 4;
m is 0 or 1;
and pharmaceutically acceptable salts thereof.

3. Composition according to claim 1 or 2, wherein X is N.

4. Composition according to claim 1, 2 or 3, wherein R₀ is chosen from H, methyl, methoxy and ethoxy.

5. Composition according to any of the preceding claims, wherein the compound (B) is:

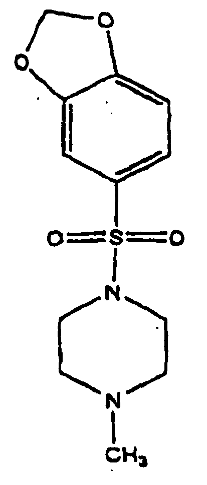

6. Composition according to any of the preceding claims, wherein R₁ and R₂ are both hydrogen, R₃ is methyl and R₄ is n-propyl.

7. Composition according to any of the preceding claims further comprising pharmaceutically acceptable adjuvant, diluent, excipient or carrier.

8. Use of a composition according to claims 1 to 7 for the production of a medicament acting as a PDE-inhibitor.

9. Use according to claim 8 for the preparation of a medicament for the treatment of erectile dysfunction.

## Patentansprüche

1. Zusammensetzung, die eine Mischung umfaßt einer Verbindung (A), dargestellt durch eine der Strukturformeln: 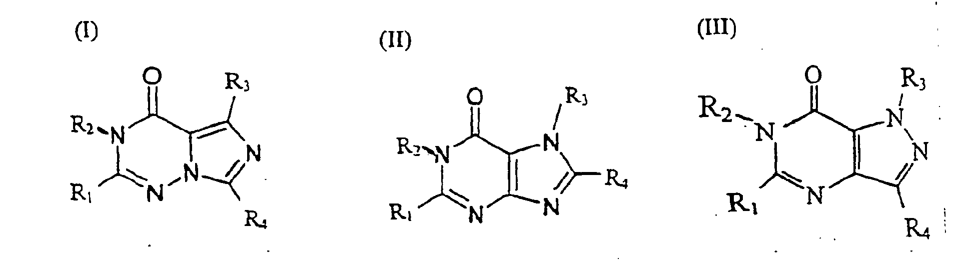 wobei R₁, R₂, R₃ und R₄ unabhängig Wasserstoff, Halogen, Hydroxyl, Amino, Alkyl, Alkenyl, Alkinyl, Cycloalkyl, Cycloalkenyl, Cycloalkinyl, Halogenalkyl, Alkylaryl, Aryl, Aralkyl, Alkoxy, Carboxy oder Heterocyclyl sind, wobei alle diese Substituenten substituiert oder unsubstituiert sind, mit Ausnahme von Formel (III), bei der R₁ Wasserstoff, C₁-C₄-Alkyl, C₁-C₄-Halogenalkyl, Piperidinomethyl, Methoxymethyl, N-Methylpiperazinomethyl, Carbethoxy, p-Chlorphenoxymethyl oder Ar-(CH₂)ₙ- ist, wobei n 0-4 ist; und wobei Ar 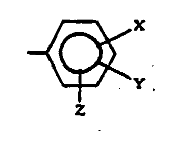 oder 2-, 3- oder 4-Pyridyl ist, wobei X, Y und Z unabhängig (1) Wasserstoff; (2) niederes Alkyl mit eins bis sechs Kohlenstoffen einschließlich; (3) Halogen; (4) Hydroxyl; (5) niederes Alkoxy mit eins bis sechs Kohlenstoffen, einschließlich; (6) Nitro; (7) Amino; (8) NR'R", wobei R' und R" jeweils unabhängig (a) Wasserstoff oder (b) niederes Alkyl mit eins bis sechs Kohlenstoffen einschließlich, optional substituiert mit (i) Amino, (ii) Morpholino oder (iii) Cycloalkyl mit fünf bis sieben Kohlenstoffen, einschließlich, sind; (9) Sulfo; oder (10) -SO₂NR'R", wobei R' und R" wie oben definiert sind, mit der Maßgabe, daß nicht alle X, Y und Z gleichzeitig Nitro, Amino oder NR'R" sein können; und wobei R₂ Wasserstoff, C₁-C₄-Alkyl, Phenyl oder 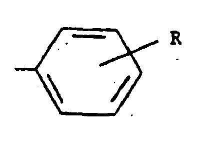 ist, wobei R ein Substituent der Gruppe bestehend aus Halogen, Methyl, Trifluormethyl und Di(C₁-C₄)alkylamino(C₁-C₄)alkyloxy ist; wenn R₃ und R₄ beide Methyl sind, und pharmazeutisch annehmbare Salze derselben,
und einer Verbindung (B), die dargestellt ist durch die Strukturformel 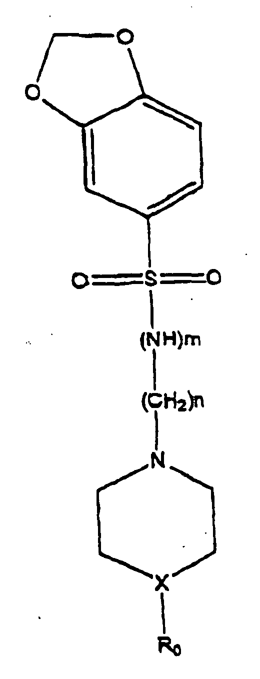 wobei
X ausgewählt ist aus N und C;
R₀ ausgewählt ist aus H, einer niederen Alkylgruppe mit 1 bis 6 Kohlenstoffatomen, einer niederen 0-Alkylgruppe mit 1 bis 6 Kohlenstoffatomen, einer niederen Aldehydgruppe mit 1 bis 6 Kohlenstoffatomen, einer Benzylgruppe, einer Phenylgruppe, einer Phenylgruppe, die mit Halogen oder O-Alkyl mit 1 bis 6 Kohlenstoffatomen substituiert ist, einer heterocyclischen Amingruppe mit 3 bis 6 Kohlenstoffatomen, einer Cycloalkylgruppe mit 3 bis 6 Kohlenstoffatomen, einer Cycloalkylgruppe mit 3 bis 6 Kohlenstoffatomen, die mit O-Alkyl mit 1 bis 6 Kohlenstoffatomen substituiert ist, und einer Furylgruppe;
n 0, 1, 2, 3 oder 4 ist;
m 0 oder 1 ist;
und pharmazeutisch annehmbare Salze derselben.

2. Zusammensetzung umfassend eine Mischung von Verbindung (III) 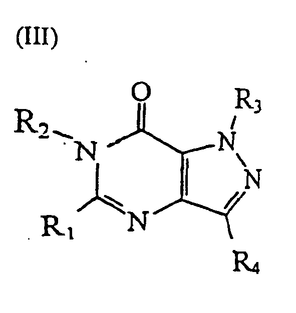 wobei R₁ und R₂ beide Wasserstoff sind, R₃ Methyl ist und R₄ n-Propyl ist,
und Verbindung (B), die durch die Strukturformel: 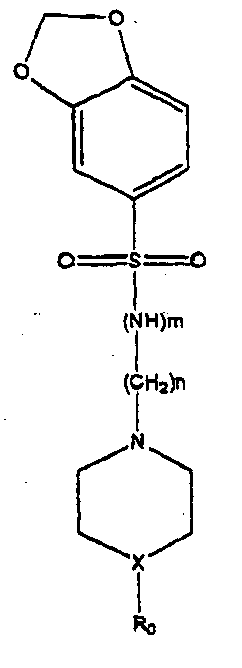 dargestellt wird, wobei
X ausgewählt ist aus N und C;
R₀ ausgewählt ist aus H, einer niederen Alkylgruppe mit 1 bis 6 Kohlenstoffatomen, einer niederen O-Alkylgruppe mit 1 bis 6 Kohlenstoffatomen, einer niederen Aldehydgruppe mit 1 bis 6 Kohlenstoffatomen, einer Benzylgruppe, einer Phenylgruppe, einer Phenylgruppe, die mit Halogen oder O-Alkyl mit 1 bis 6 Kohlenstoffatomen substituiert ist, einer heterocyclischen Amingruppe mit 3 bis 6 Kohlenstoffatomen, einer Cycloalkylgruppe mit 3 bis 6 Kohlenstoffatomen, einer Cycloalkylgruppe mit 3 bis 6 Kohlenstoffatomen, die mit O-Alkyl mit 1 bis 6 Kohlenstoffatomen substituiert ist, und einer Furylgruppe;
n 0, 1, 2, 3 oder 4 ist;
m 0 oder 1 ist;
und pharmazeutisch annehmbare Salze derselben.

3. Zusammensetzung nach Anspruch 1 oder 2, wobei X N ist.

4. Zusammensetzung nach Anspruch 1, 2 oder 3, wobei R₀ ausgewählt ist aus H, Methyl, Methoxy und Ethoxy.

5. Zusammensetzung nach einem der vorangehenden Ansprüche, wobei die Verbindung (B) die folgende ist:

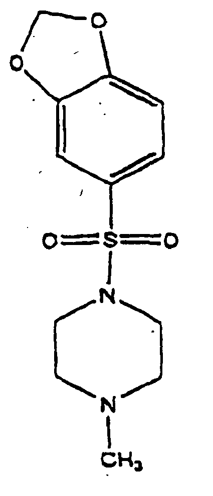

6. Zusammensetzung nach einem der vorangehenden Ansprüche, wobei R₁ und R₂ beide Wasserstoff sind, R₃ Methyl ist und R₄ n-Propyl ist.

7. Zusammensetzung nach einem der vorangehenden Ansprüche, weiter umfassend einen pharmazeutisch annehmbaren Hilfsstoff, Verdünnungsmittel, Vehikel oder Träger.

8. Verwendung einer Zusammensetzung nach den Ansprüchen 1 bis 7 für die Herstellung eines Medikaments, das als ein PDE-Inhibitor wirkt.

9. Verwendung nach Anspruch 8 für die Herstellung eines Medikaments für die Behandlung erektiler Dysfunktion.

## Revendications

1. Composition comprenant un mélange du composé (A) représenté par l'une des formules structurelles : 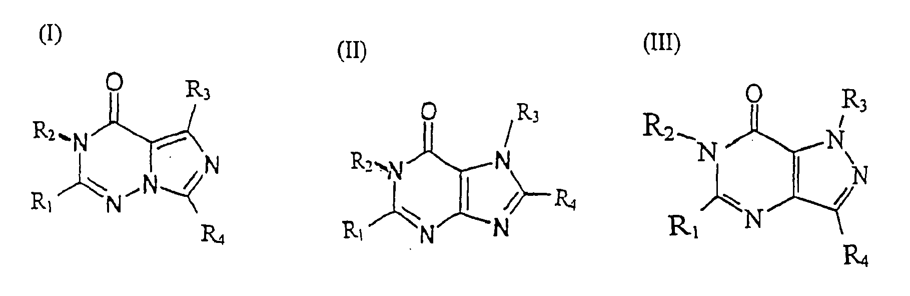 dans lesquelles R₁, R₂, R₃ et R₄ représentent indépendamment un atome d'hydrogène, un atome d'halogène, un groupe hydroxyle, un groupe amino, un groupe alkyle, un groupe alcényle, un groupe alcynyle, un groupe cycloalkyle, un groupe cyclo-alcényle, un groupe cycloalcynyle, un groupe halogénoalkyle, un groupe alkylaryle, un groupe aryle, un groupe aralkyle, un groupe alcoxy, un groupe carboxy ou un groupe hétérocyclyle, tous ces substituants étant substitués ou non substitués, à l'exception de ceux de la formule (III) dans laquelle R₁ représente un atome d'hydrogène, un groupe alkyle en C₁-C₄, un groupe halogénoalkyle en C₁-C₄, un groupe pipéridinométhyle, un groupe méthoxyméthyle, un groupe N-méthylpipérazinométhyle, un groupe carbéthoxy, un groupe p-chlorophénoxyméthyle ou Ar-(CH₂)ₙ-, où n vaut de 0 à 4 ; et Ar représente 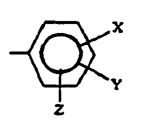 ou un groupe 2, 3, ou 4-pyridyle, où X, Y et Z représentent indépendamment (1) un atome d'hydrogène ; (2) un groupe alkyle inférieur de 1 à 6 atomes de carbone inclus ; (3) un atome d'halogène ; (4) un groupe hydroxyle ; (5) un groupe alcoxy inférieur de 1 à 6 atomes de carbone inclus ; (6) un groupe nitro ; (7) un groupe amino ; (8) NR'R" où R' et R" représentent chacun indépendamment (a) un atome d'hydrogène ou (b) un groupe alkyle inférieur de 1 à 6 atomes de carbone inclus, éventuellement substitué par (i) un groupe amino, (ii) un groupe morpholino ou (iii) un groupe cycloalkyle de 5 à 7 atomes de carbone inclus ; (9) un groupe sulfo ; ou (10) - SO₂NR'R" où R' et R" sont tels que défini ci-dessus à condition que pas tous les groupes X, Y et Z puissent être un groupe nitro, un groupe amino, ou NR'R" en même temps ; et R₂ représente un atome d'hydrogène, un groupe alkyle en C₁-C₄, un groupe phényle ou 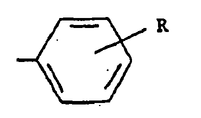 où R représente un substituant du groupe constitué par un atome d'halogène, un groupe méthyle, un groupe trifluoro-méthyle et un groupe di (alkyle en C₁-C₄) amino (alkyloxy en C₁-C₄) ; lorsque R₃ et R₄ sont tous les deux un groupe méthyle,
et des sels pharmaceutiquement de celui-ci,
et du composé (B) représenté par la formule structurelle : 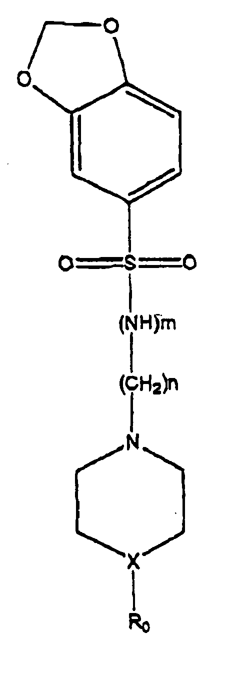 dans laquelle
X est choisi parmi N et C ;
R₀ est choisi parmi H, un groupe alkyle inférieur ayant de 1 à 6 atomes de carbone, un groupe O-alkyle inférieur ayant de 1 à 6 atomes de carbone, un groupe aldéhyde inférieur ayant de 1 à 6 atomes de carbone, un groupe benzyle, un groupe phényle, un groupe phényle substitué par un atome d'halogène ou un groupe O-alkyle ayant de 1 à 6 atomes de carbone, un groupe amine hétérocyclique ayant de 3 à 6 atomes de carbone, un groupe cycloalkyle ayant de 3 à 6 atomes de carbone, un groupe cycloalkyle ayant de 3 à 6 atomes de carbone substitué par un groupe O-alkyle ayant de 1 à 6 atomes de carbone, et un groupe furyle ;
n vaut 0, 1, 2, 3 ou 4 ;
m vaut 0 ou 1 ;
et des sels pharmaceutiquement acceptables de celui-ci.

2. Composition comprenant un mélange du composé (III) 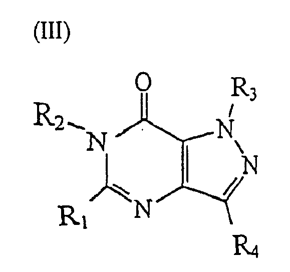 dans lequel R₁ et R₂ représentent tous les deux un atome d'hydrogène, R₃ représente un groupe méthyle, et R₄ représente un groupe n-propyle,
et du composé (B) représenté par la formule structurelle : 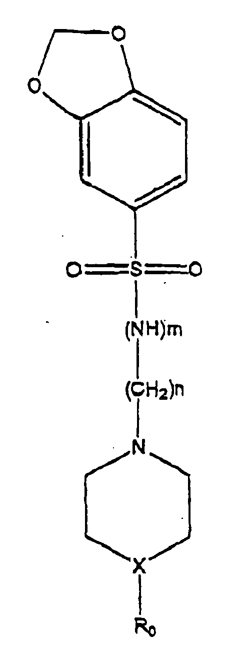 dans laquelle
X est choisi parmi N et C ;
R₀ est choisi parmi H, un groupe alkyle inférieur ayant de 1 à 6 atomes de carbone, un groupe O-alkyle inférieur ayant de 1 à 6 atomes de carbone, un groupe aldéhyde inférieur ayant de 1 à 6 atomes de carbone, un groupe benzyle, un groupe phényle, un groupe phényle substitué par un atome d'halogène ou un groupe O-alkyle ayant de 1 à 6 atomes de carbone, un groupe amine hétérocyclique ayant de 3 à 6 atomes de carbone, un groupe cycloalkyle ayant de 3 à 6 atomes de carbone, un groupe cycloalkyle ayant de 3 à 6 atomes de carbone substitué par un groupe O-alkyle ayant de 1 à 6 atomes de carbone, et un groupe furyle ;
et des sels pharmaceutiquement acceptables de celui-ci.

3. Composition selon la revendication 1 ou 2, dans laquelle X représente N.

4. Composition selon la revendication 1, 2 ou 3, dans laquelle R₀ est choisi parmi H, un groupe méthyle, un groupe méthoxy et un groupe éthoxy.

5. Composition selon l'une quelconque des revendications précédentes, dans laquelle le composé (B) est :

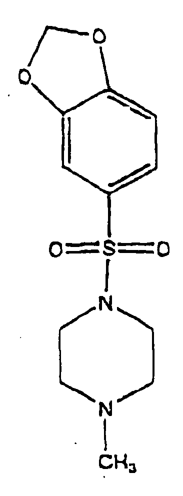

6. Composition selon l'une quelconque des revendications précédentes, dans laquelle R₁ et R₂ représentent tous les deux un atome d'hydrogène, R₃ représente un groupe méthyle et R₄ représente un groupe n-propyle.

7. Composition selon l'une quelconque des revendications précédentes, comprenant en outre un adjuvant, un diluant, un excipient ou un support pharmaceutiquement acceptable.

8. Utilisation d'une composition selon les revendications 1 à 7, pour la préparation d'un médicament agissant en tant qu'inhibiteur de la DPE.

9. Utilisation selon la revendication 8, pour la préparation d'un médicament destiné au traitement d'un dysfonctionnement érectile.
